Europäisches Patentamt

European Patent Office

Office européen des brevets

(19)

(11) Publication number : **0 633 462 A2**

## (12) EUROPEAN PATENT APPLICATION

(21) Application number : **94304077.4**

(22) Date of filing : **07.06.94**

(51) Int. Cl.$^6$ : **G01N 15/14**

(30) Priority : **08.06.93 US 74065**
**07.03.94 US 207302**

(43) Date of publication of application :
**11.01.95 Bulletin 95/02**

(84) Designated Contracting States :
**BE CH DE DK ES FR GB IT LI LU NL SE**

(71) Applicant : **ORTHO DIAGNOSTIC SYSTEMS INC.**
**U.S. Route 202**
**Raritan New Jersey 08869 (US)**

(72) Inventor : **Mercolino, Thomas J.**
**121 Lambertville,**
**Headquarters Road**
**Stockton, New Jersey 08559 (US)**
Inventor : **Meyer, Eric J.**
**Route 523**
**Flemington, New Jersey 08822 (US)**
Inventor : **Petrou, George V.**
**119 Hazlewood Avenue**
**Metuchen, New Jersey 08840 (US)**
Inventor : **Griffis, Mark D.**
**432 Montgomery Street**
**Highland Park, New Jersey 08904 (US)**
Inventor : **DeChirico, Nino**
**273 Spruce Court**
**Flemington, New Jersey 08822 (US)**

(74) Representative : **Mercer, Christopher Paul et al**
**Carpmaels & Ransford**
**43, Bloomsbury Square**
**London WC1A 2RA (GB)**

(54) **Three-color flow cytometry with automatic gating function.**

(57)   A flow cytometry based system is provided which uses a three-color reagent panel in conjunction with a cytometer which permits direct absolute cell counts. Additionally, a system for automatic accurate selection of subpopulations is provided.

FIG 7A
Graph 1
FW-SC
RT-SC

EP 0 633 462 A2

A user guide titled "ImmunoCount User Guide," distributed by Ortho Diagnostics Systems, Inc., Raritan, New Jersey and relating to the invention disclosed herein is incorporated herein by reference and made a part of this application.

## Background of the Invention

Worldwide, the number of individuals infected with the Human Immunodeficiency Virus (HIV) continues to rise. The World Health Organization estimates over 30-40 million HIV+ cases by the year 2000. Progression of HIV disease to Acquired Immunodeficiency Syndrome (AIDS) now takes, on average, a half-time of approximately 10 years in developed countries. These two factors combined result in an ever increasing need for better tools to monitor HIV disease progression.

Understanding of the clinically important factors for monitoring HIV infection has increased significantly in the past few years. This increased understanding has lead the Centers for Disease Control (CDC) to re-evaluate how the stages of HIV disease progression are to be classified. The CDC report concludes that absolute CD4$^+$ T-Lymphocyte count (absolute CD4$^+$ count) is the most important marker available to clinicians for staging purposes. Further, the report proposes that AIDS may be defined solely on the basis of absolute CD4$^+$ count when it falls below 200. Thus, the CDC has expanded the previous definition of AIDS beyond clinical presentation of "indicator diseases".

Finally, the CDC report recommended that physicians use a patient's absolute CD4$^+$ count to decide when to begin anti-retroviral and prophylactic therapies. Conflicting reports have been published concerning the benefits to be gained from prophylactic azidothymidine (AZT) therapy in individuals with CD4$^+$ T-lymphocyte counts less than 500/uL. However, in clinical practice, initiation of AZT and other therapies is typically based upon absolute CD4$^+$ count. Perhaps the most successful of the prophylactic therapies has been against Pneumocystis carini pneumonia (PCP), the most common opportunistic infection in AIDS. PCP prophylaxis is usually begun at an absolute CD4$^+$ count below 200. Proper timing of prophylactic therapy is a major reason people infected with HIV are living longer, healthier lives than in the early days of the pandemic. Thus, resulting from both the CDC recommendation and accepted clinical practice, the absolute CD4$^+$ count has become the most important criterion in the laboratory determination of AIDS.

HIV has been strongly implicated as the causative agent of AIDS. The virus recognizes and binds to "helper" T-lymphocytes via the CD4 antigen expressed on their surface. HIV eventually compromises immunity presumably by mediating the elimination of CD4$^+$ T-lymphocytes. Since the CD4$^+$ T-lymphocyte is known to play an important role in the human immune response, the decrease in the absolute number of circulating CD4$^+$ T-lymphocytes following HIV infection has been shown to correlate closely with the development of HIV-related conditions. These conditions include pronounced immunodeficiency, opportunistic infections, and cancers.

CD4 and other cell-surface antigens are identified by monoclonal antibodies. These antibodies are grouped into "clusters of differentiation" (CD) depending upon the antigen against which they are directed. Specific lymphocyte subsets may be identified by labeling cells with monoclonal antibodies directed against these CD antigens. Conjugating each antibody type with a dye of distinguishable fluorescence allows individual cells to be classified based upon the binding of one or more antibodies in a simultaneous assay. This type of analysis constitutes a direct identification of specific cell types as contrasted with indirect methods such as ELISA which require more controls, and can potentially result in a higher chance of false identification.

Flow cytometers are widely used to enumerate cell subsets based upon the binding of fluorescent monoclonal antibodies. These instruments are designed to cause cells in suspension to travel in single-file through a laser beam for interrogation. Cells are then classified by the way in which they scatter the incident laser light, which provides information on the size and internal granularity of the cells. Further, any fluorescent light they may emit permits the detection of specific antibody binding, and thus subsequent cell-subset identification. A general description of standard flow cytometry apparatus and methods is provided in U.S. Patent No. 4,284,412 to Hoffman and Hansen entitled "Method and Apparatus for Automated Identification and Enumeration of Specified Blood Cell Subclasses" and incorporated by reference herein.

Many laboratories use two tests to derive the CD4 count. A fluorescence flow cytometer is used to measure the fraction of lymphocytes that are CD4$^+$ and a hematology instrument is used to count the number of lymphocytes per unit volume of blood. The product of the two measurements gives the CD4$^+$ count. By using the two separate tests, the error is multiplied, making the total variability much greater than for each test alone. Typically, the tests are done in different parts of the laboratory. It is also typical for each test to be done using two different tubes of blood, each collected in a different type of anticoagulant. The samples may not be handled identically, or be run at the same time. These factors further contribute to the error of the determination of CD4$^+$ count. Finally, and probably most critically, the hematology portion of the test lacks standardization. Every manufacturer's hematology instrument uses a different technology for determining the lymphocyte count. This can result in a difference in lymphocyte count of 30% or greater between labs that use different hematology instruments.

Other laboratory tests have been proposed for monitoring the progression of HIV disease, including $CD8^+$ T-lymphocyte count, serum neopterin, beta-2 microglobulin, HIV p24 antigen, soluble interleukin-2 receptors, and immunoglobulin A. None of these cellular or serological markers have been as strongly predictive of disease progression, nor as specific for HIV-related immunosuppression as absolute $CD4^+$ T-lymphocyte count. The absolute $CD4^+$ count remains the essential laboratory measurement for proper monitoring, staging and clinical management of the HIV infected patient.

The current CDC recommended panel for HIV immunophenotyping consists of a six reagent, two-color panel consisting of the following reagents in conjunction with an absolute lymphocyte determination:

Hematology or cytometer derived absolute count sample
CD45/CD14
CD3/CD4
CD3/CD8
CD3/CD19
CD3/CD16 &/or CD56

These reagents are used in a variety of systems and analysis schemes, each having its own advantages and disadvantages. However, inherent in all are several sources of error. The current CDC recommended panel requires that a patient sample be separated into six aliquots for an analysis on the flow cytometer to obtain the information necessary for a complete T-lymphocyte analysis. Furthermore, this reagent format does not permit direct absolute counts of subsets of cell types. Subpopulations of CD4 T-lymphocytes, for example, are enumerated by determining the ratio of CD4 cells to total lymphocytes. Any error in the initial total lymphocyte count is reflected in the CD4 count.

Current algorithms for automatic cell subpopulation detection in flow cytometry are cytogram specific and cell type specific. Such algorithms typically can only be applied on a forward vs. right scatter cytogram and can only detect lymphocyte populations. Thus, it is an object of the present invention to provide a method for detecting cell subpopulations which can be applied to any cytogram, independent of the parameters which define the cytogram. It is also an object of the invention to provide such a method which is capable of detecting any type of subpopulation represented in a cytogram.

The technology for generating absolute $CD4^+$ counts has changed little since before the HIV pandemic. The present methods being used to measure absolute $CD4^+$ counts reflect little change over the flow cytometry methods in use in 1980. In particular, current flow cytometry methods, by themselves, generally do not enable absolute cell counts, and a separate analysis, such as a hematology test, is required to determine cell counts. It is an object of the present invention to provide an apparatus and method for obtaining direct absolute cell counts in a flow cytometry system.

Summary of the Invention

The system of the present invention comprises a cell analysis instrument and method based upon flow cytometry, in conjunction with three components: (1) flow cytometer calibrators; (2) cell labeling reagents; and (3) software for automatically selecting cell populations. When used together, systems in accordance with the present invention provide precise and accurate absolute counts of total cells and subset populations, including absolute $CD4^+$ count. The invention encompasses two main aspects. Firstly, a flow cytometry instrument capable of metering a known number of cells per unit volume is used in conjunction with a reagent panel formatted to render detectable specific cells of interest, to permit direct absolute counts of total cells and cell subpopulations. Secondly, a method for identifying and selecting a subpopulation of data points accurately defines the gate region which encompasses the subpopulation of interest, and excludes data points which are not part of the subpopulation. Embodiments disclosed herein are consistent with the recommendations of the CDC. All data and patient reports are generated by completely automated software. Compared to other systems, the present system provides sample preparation which is faster and safer; fewer steps are required and centrifugation is completely eliminated. The data produced are of higher quality than in other systems. The overall result is a savings of time, reduced expense, and less retesting.

The present invention provides for automatic cell subpopulation detection that can be applied on any cytogram and can detect any cell subpopulation on that cytogram. Because the algorithm is general, it can be applied in HIV immunophenotyping, leukemia and lymphoma detection and other areas of research and clinical diagnosis where flow cytometric techniques are used. The present invention incorporates advances in immunology, reagents, instrumentation and automation into an improved system for absolute cell subset analysis.

The present invention utilizes a flow cytometer which performs absolute cell-subset counts without the need for additional tests, such as hematology. The result is accurate and reproducible injection of sample at a known rate past the laser beam. Since the time of sample injection may be easily measured, the volume of sample analyzed is known. This principle has been shown to be accurate and reproducible in determining absolute total lymphocyte and $CD4^+$ T-lymphocyte levels in peripheral blood. When used in conjunction with the associated reagents and software of the invention, it is possible to: (1) calibrate, verify, and quality control the absolute count function of the flow cytometer, as well as to cross-calibrate individual instruments; (2) obtain all the information provided by the CDC recommended panel, more quickly and safely with less steps than in other systems; (3) provide for patient sample, reagent and system quality control; and (4) to obtain automated data analysis immediately following sample acquisition, while providing for instrument and reagent quality control. Additionally, the instrument design allows function without a significant possibility of aerosol being produced from potentially hazardous samples. When used with an optional automated sample handling unit, the system further reduces the chance of laboratory contamination; once samples are prepared, all additional manipulation and analysis is completely automated in an entirely isolated environment. The system design allows laboratories to routinely acquire accurate and reproducible absolute CD4 counts in a manner superior to other means for measuring CD4, which are more prone to variability.

BRIEF DESCRIPTION OF THE FIGURES

Figure 1 is a diagrammatic representation of the optical system of a flow cytometer of the present invention using 3-color detection;
Figures 2 and 3 are a representation of analysis of sample plus control reagent;
Figure 4 is a representation of analysis of sample plus labelled CD4/CD8/CD3 reagent;
Figures 5 and 6 are a representation of analysis of sample plus labelled CD16/CDI9/CD3 reagent;
Figures 7A-7F are further representations of analysis of the control sample;
Figures 8A-8D are further representations of analysis of sample plus labelled CD4/CD8/CD3 reagent;
Figures 9A-9F are further representations of analysis of sample plus labelled CD16/CD19/CD3 reagent;
Figure 9G illustrates an alternate analysis that may be made of the sample plus labelled CD16/CD19/CD3 reagent; and
Figure 10 shows data that may be generated in the operation of this invention.

## DETAILED DESCRIPTION OF THE INVENTION

### Flow Cytometer Capable of Direct, Absolute Cell Counts

The flow cytometer component of the present invention is capable of metering a known volume of sample at a known rate past the laser. Its syringe-drive fluidics system uses syringes that are driven by precision stepping motors. This is accomplished by the use of a calibration kit which is used to calibrate and quality control the absolute count function of the flow cytometer using a system of microparticle suspensions containing a known number of particles per unit volume. A well-known and widely available flow cytometer and calibration kit useful for this embodiment are available from Ortho Diagnostic Systems Inc., Raritan, New Jersey, under the trade name CYTORONABSOLUTE™. A users' manual which describes in detail the instrument and its function is incorporated by reference herein, and is also available from Ortho. Microparticle calibration offers greater convenience over other protocols since there is no need for separate hematological instrumentation. Conversely, if hematology instrumentation were used in calibration, the accuracy of the flow cytometer would be completely dependent on the accuracy of the hematology instrumentation. Absolute lymphocyte counts performed using hematological methods have been shown to vary by as much as 40% depending on which hematological instrument is used. However, the number per unit volume of stable microparticles in suspension used in the present system are well known.

Calibration of the flow cytometer is entirely software-driven. The instrument samples the suspension, and counts the particles up to a predetermined number, while also recording the time required to make the count. From these data, and from the concentration of the calibrators, the instrument calculates the time required to deliver a known volume of sample. Thus, whenever the flow cytometer delivers a sample for that calibrated amount of time, the number of events are measured per known volume. To assure the accuracy of the calibration process, the calibration suspension requires no user intervention prior to sampling. Calibration is thereby made independent of the user's precision pipetting or other dilution, and the risks associated with introduction of a pipette in the calibrator suspension is eliminated.

The calibration kit also provides for daily verification of instrument calibration. Three additional suspensions of particles at known number per unit volume are also supplied. The number/volume of these suspensions correspond roughly to values expected from patients with low, normal and high white blood cell counts, respectively. If the concentration values determined by the flow cytometer are within specification, the calibration is verified. Keeping a record of these verification results monitor performance over time and document quality assurance of the instrument's count function.

As a result, the data generated with respect to any individual cell type being analyzed, represents an absolute count of those cells.

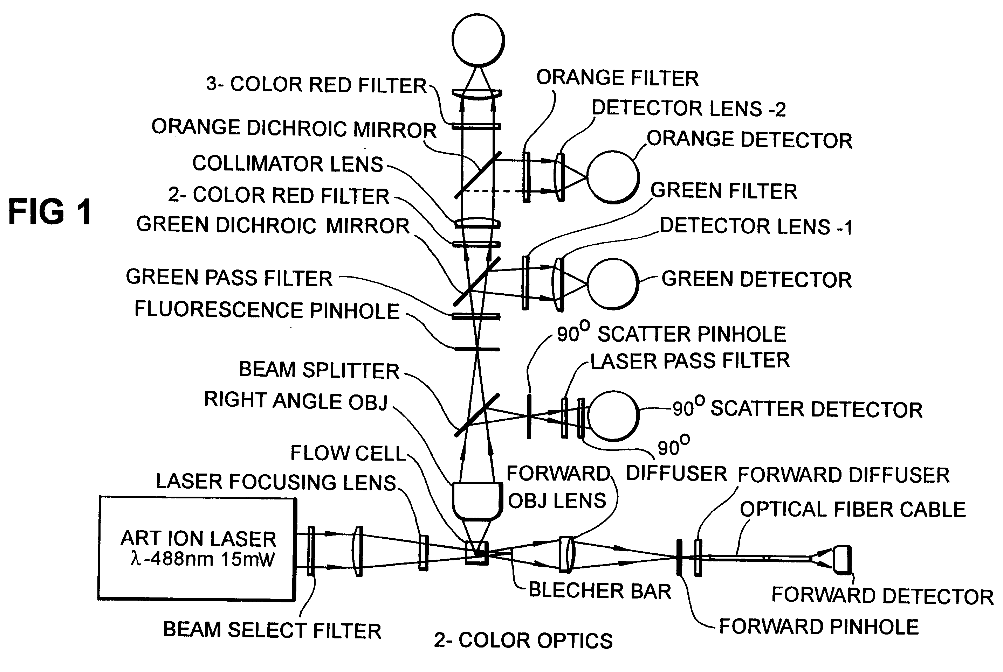

The optic system of a flow cytometer of a preferred embodiment of the invention is shown diagrammatically in Figure 1. The instrument is configured to detect forward scatter, right angle scatter, and three colors of fluorescence, namely green, orange and red. An argon ion laser emits a laser beam through the beam select filter and laser focusing lens from which it is directed on to sample dispensed through the flow cell. Forward-scattered light is directed through the forward objected lens, the forward pin hole, the forward diffuser, and into and optical fiber cable, from which it is directed to the forward detector. Light which is scattered by the sample is directed through the right angle objective lens and a beam splitter where part of the signal is detected through a 90° scatter pin hole, a laser pass filter, a 90° diffuser, and then is detected by the 90° scatter detector. The remainder of the scatter light passes through the beam splitter, a fluorescence pinhole, a green pass filter, and then to a green pass filter where part of the light is deflected through a green filter, and a detector lens to the green detector. The remaining light passes through a columnate lens through and orange dichroic mirror where part of the light is deflected through an orange filter and a detector lens to the orange detector. The remaining light proceeds through a three color red filter and a detector lens to the red detector. The optic system shown diagrammatically if Figure 1 represents the system used in CYTORONABSOLUTE™ and is presented to illustrate one embodiment of the flow cytometer of the invention, those skilled in the art will recognize that a flow cytometer optic system may be configured in various ways to achieve the same results.

Three Color Reagent Panel

The reagents used in the present invention are an immunophenotyping system using specifically formatted 3-color monoclonal antibody reagents. When used in a preferred embodiment, these provide for the identification and enumeration of T, B and NK cell lymphocyte subsets, as well as total lymphocyte counts, using only three sample tubes. This reagent system represents a reduction in the amount of tubes required to obtain the same amount of data as obtained from the CDC recommended two-color panel using six tubes. Additionally, the reagents distinguish between specific and non-specific antibody/cell interactions, allow for spectral compensation of the instrument, correct for inherent sources of error, and provide for the quality control of the patient sample, reagent, and system.

When formatted to analyze lymphocytes, the system comprises three reagents: an isotypic control, a T-helper/T-cytotoxic suppressor/total T-cell reagent, and a NK/B/T-cell reagent. Each reagent consists of three monoclonal antibodies, each with different specificities, each conjugated to a different fluorochrome. The fluorochromes chosen must either have emission spectrums which are non-overlapping or whose overlap can be electronically or mathematically compensated. If spectral compensation is necessary, the antibody-fluorochrome conjugate combinations must be chosen so that compensation can be checked and adjusted using either one or both of the two positive staining reagents. Identical conjugates of non-leukocyte specific mouse monoclonals of the same isotype as the positive specificities are also necessary for use as negative staining controls.

A specific example of antibody-conjugate combinations to produce a reagent panel with the desired characteristics for analyzing lymphocytes are:

1. Control IgGl-Fluorescein Isothiocyanate (FITC) + IgG2a-FITC/IgGl-Phycoerythrin (PE) + IgG2a-PE/IgG2a-Carbonocyanine-5/Phycoerythrin Tandem (CyP)
2. CD4-FITC/CD8-PE/CD3-CyP
3. CD16-FITC/CD19-PE/CD3-CyP

These fluorochromes all are excited by the 488 nm emission line from an argon laser, and permit their fluorescence detection in the green (FITC), orange (PE), and red (CyP) regions of the spectrum, respectively. Spectral compensation is accomplished electronically.

The assay format used must produce staining of all lymphocyte subsets with sufficient signal to noise S/N ratios. Typically, the red cells must be lysed, and the leukocyte viability and light scatter characteristics must be maintained to allow identification of the leukocyte populations. Acceptable resolution between cell populations and debris is also necessary. Any physical manipulation which may cause random or specific cell loss within the WBC subsets cannot be present in the procedure.

Each reagent provides for the following when used on flow cytometry:

Isotypic control - By gating on the lymphocyte population of cells as determined by light scatter, the green and orange fluorescence of that population may be examined. This protocol allows for an absolute count of the lymphocytes. Statistical regions also may be set with this reagent to delineate levels of non-specific binding and autofluorescence. These phenomena are thereby eliminated in measurements of negative and positive staining with subsequent reagents.

CD4/CD8/CD3 - Based on light scatter parameters, a lymphocyte population may be obtained. Plotting red fluorescence against right-angle light scatter permits the CD3$^+$ cells to be distinguished from the other populations, and an absolute count to be obtained. Examining this CD3$^+$ population further for green and orange fluorescence permits the identification of CD4$^+$ and CD8$^+$ populations as well as an absolute count of each.

CD16/CD19/CD3 - Again an absolute lymphocyte and absolute T-cell count may be determined using the methods above. Examination of the orange fluorescence versus right angle scattering permits measurement of the absolute B-cell count. Examination of the green fluorescence versus right angle scattering allows a determination of the absolute count of CD16$^+$ cells, and examination of the red fluorescence of the CD16$^+$ population enables the detection of an absolute count of CD16$^+$CD3$^+$ cells. Subtracting the count of cells that are CD16$^+$CD3$^+$ from the count of cells that are CD16$^+$ produces a count of the number of cells that are CD16$^+$CD3$^-$. These latter cells are the NK cells.

Using this complete set of reagents for each patient sample permits the determination of triplicate direct absolute total lymphocyte count, duplicate direct absolute CD4$^+$ T-cell count, absolute NK cell count, and absolute B cell count. A positive, immunological determination of the absolute lymphocyte count can also be determined from the absolute T cell, NK cell, and B cell counts. This latter method is free from potential RBC, monocyte and platelet contamination, which often compromise determinations based exclusively upon light scatter parameters. The lymphocyte population may be determined more positively than with a CD45/CD14 reagent. Antibody against CD45 is not specific for lymphocytes and only about 80% of contaminating monocytes would be expected to bear CD14. In contrast, the present reagents are used with immuno-scatter gating to positively identify the three subsets of cells that comprise lymphocytes. The lymphocyte absolute count is quality controlled by comparing the sum of NK$^+$ and B$^+$ T-cell subsets to the light scatter-gated lymphocyte determination. The present reagents use anti-CD16 instead of anti-CD56 to identify NK cells; compared to CD56, CD16 antigen expression on NK cells remains relatively constant during the course of HIV infection.

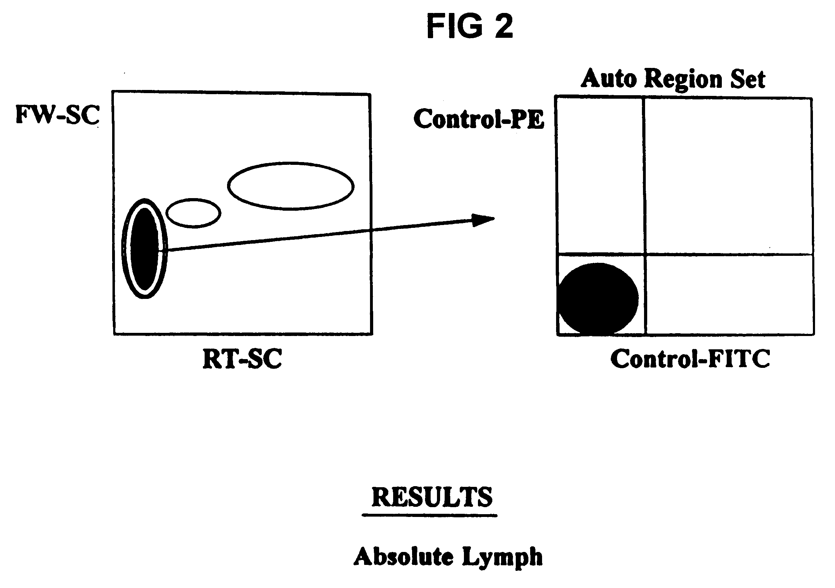

A data acquisition and analysis scheme is diagrammatically represented in the attached Figures 2-6, using the three tube reagent panel given as an example above. The results obtained from each tube are also listed.

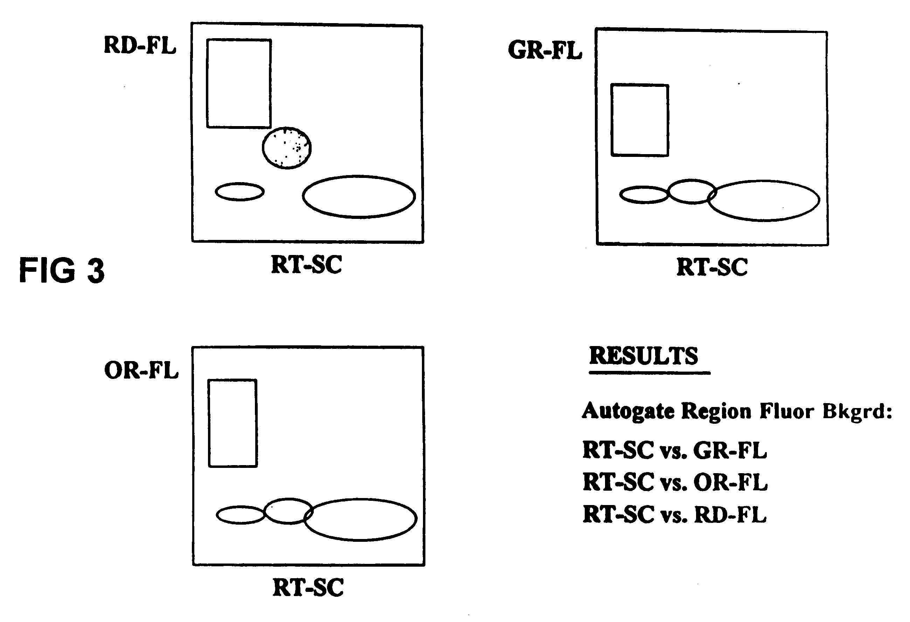

The control stained patient sample is run first. A light scatter lymphocyte gate is either manually or, in the preferred embodiment, automatically set around the lymphocyte population, and an absolute lymphocyte count is determined (Figure 2). The green and orange fluorescent distributions for the negative control stained lymphocytes are then plotted on a green vs. orange fluorescence cytogram. Statistical regions are set in order to delineate double negative, orange only, green only, and green + orange stained cells from one another in the other two tubes of the assay (Figure 2). The non-specific fluorescent background in each of the three immuno-scatter gate search regions to be used in the following two tubes, is also determined (Figure 3).

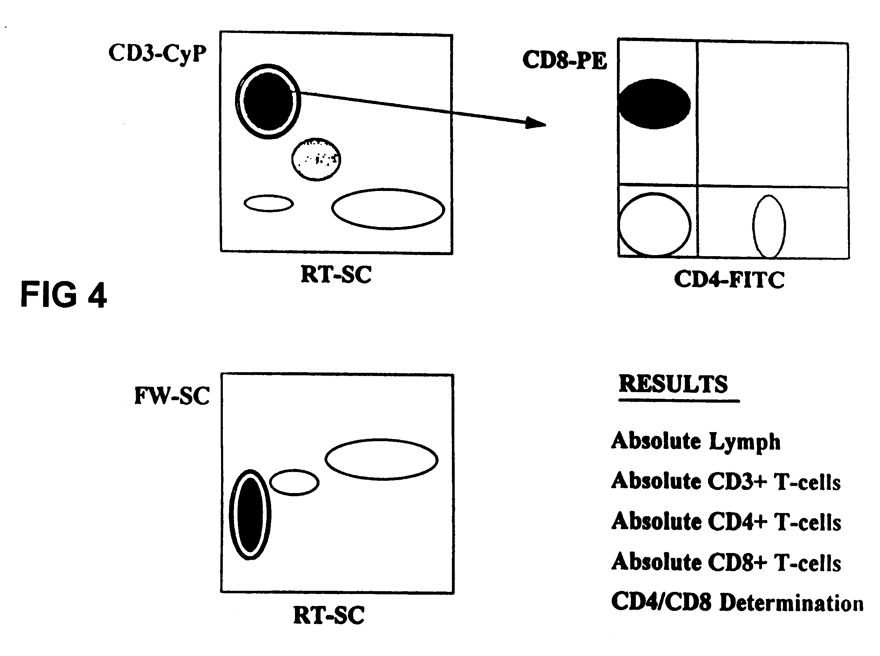

The CD4/CD8/CD3 stained sample is run next (Figure 4). An absolute lymphocyte count is determined using a light scatter gate, and an "immuno-scatter" gate is set around the CD3$^+$ events, resulting in another absolute T-cell determination. The CD3$^+$ cells are then plotted on the green vs. orange (CD4 vs. CD8) fluorescence cytogram, resulting in absolute CD4 and CD8 count determinations. A CD4 to CD8 ratio could be calculated at this point, as well.

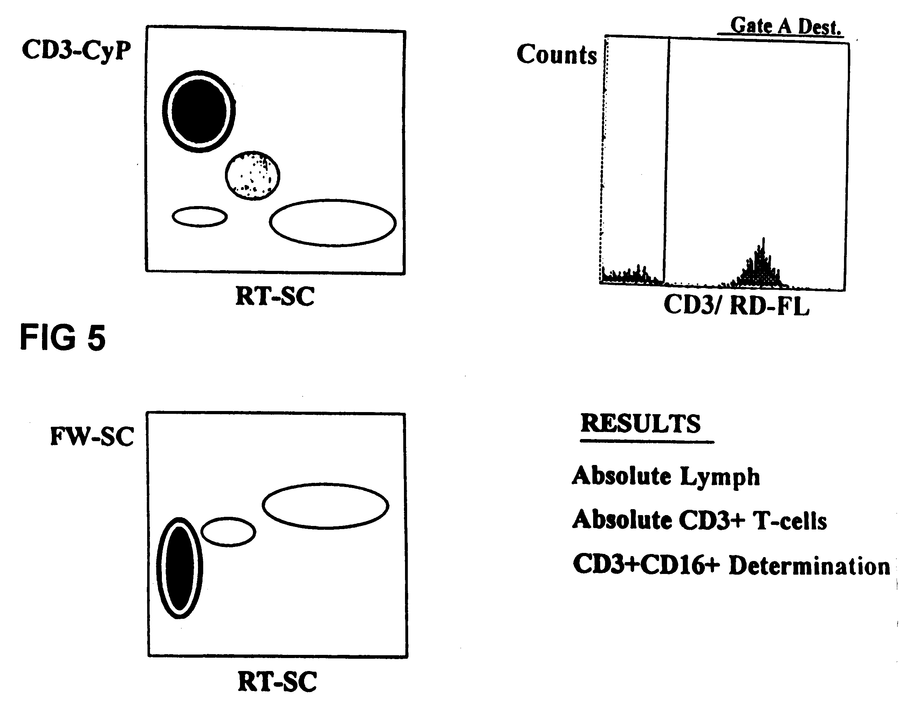

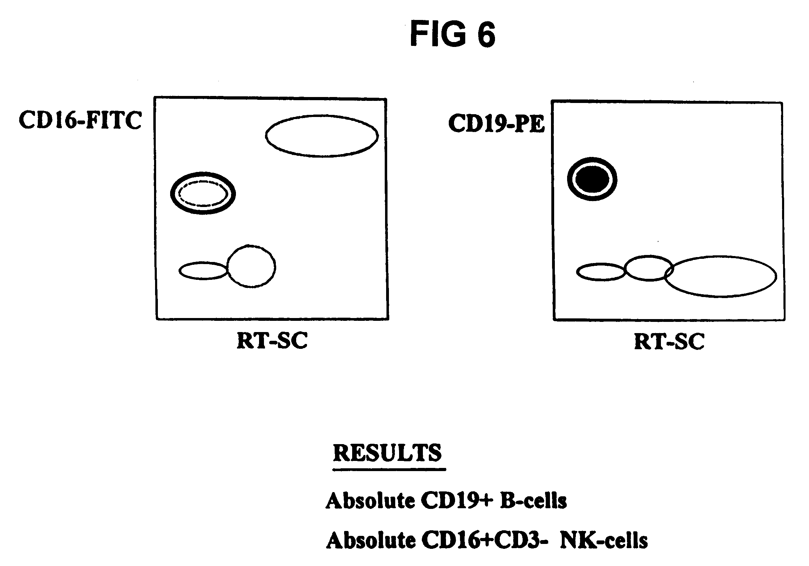

Finally, the CD16/CD19/CD3 sample is run. Again, an absolute lymphocyte count is determined using a light scatter gate, and an immuno-scatter gate is set around the CD3$^+$ events, resulting in another absolute T-cell determination (Figure 5). The absolute count of the CD16$^+$ and CD19$^+$ events are determined using two immuno-scatter gates (Figure 6). Examination of the red fluorescence of the CD16$^+$ population permits the detection of the absolute count of CD16$^+$CD3$^+$ cells (Figure 5). Although it is rare for T-cells to express CD16, if such T-cells are present they must be subtracted from the absolute CD16$^+$ count determination in order to determine accurately the number of NK-cells, all of which are CD16$^+$CD3$^-$. An absolute count of the NK cells is therefore determined by subtracting the number of cells that are CD16$^+$CD3$^+$ from the number of cells that are CD16$^+$.

A summary of the results and data corrections, calculations, and cross-checks obtained from a sample are listed below.

RESULTS AND CROSS-CHECKS

Tube Specific Raw Data:
      Control Tube:
            Absolute Lymph:          1262
            Autogate Fluor Bkgrd:    0
      CD4/CD8/CD3 Tube:
            Absolute Lymph:          1021
            Absolute CD3:            753
            Absolute CD4+ T-cells:  543
            Absolute CD8+ T-cells:  173
      NK/B/T Tube
            Absolute Lymph:          1133
            Absolute CD3:            809
            Absolute CD19+ B-cells:  122
            Absolute CD3+CD16+ Lymphs: 2
            Absolute CD16+ Lymphs:  277


Cross-checked/Corrected data
        Mean Absolute Lymph:      1139
        Autogate Fluor Bkgrd Corr:  none
        Sum of CD3+CD19+CD16$^+$CD3$^-$:  1206
        X-checked Mean Abs Lymph:  1173
        Mean Absolute CD3:        781
        CD16$^+$CD3$^-$ NK-cells:     275

| REPORTED RESULTS | |
|---|---|
| X-Checked Mean Absolute Lymph: | 1173 cells/ul |
| Mean Absolute CD3: | 781 cells/ul |
| Absolute CD4$^+$ T-cells | 543 cells/ul |
| CD4/CD8 Determination: | 3.14 |
| CD4$^+$ T-cells as % of Mean Lymphs | 46.3% |
| CD8+ T-cells as % of Mean Lymphs | 14.8% |
| CD3+ T-cells as % of Mean Lymphs | 66.6% |
| CD19+ B-cells as % of Mean Lymphs | 10.4 % |
| CD16+CD3- NK-cells as % of Mean Lymphs | 23.4% |

If autogate fluorescent background, or CD3+ CD16$^+$ events were detected, they can be subtracted from the appropriate absolute count determinations. In addition, within each patient sample, triplicate absolute lymphocyte counts, and duplicate absolute CD3 determinations were made which can be compared, a confidence level determined, and if applicable, their mean taken. The sum of the CD3+, CD19+, and CD16+ CD3- events can be calculated. This provides an absolute lymphocyte count derived by a method which does not include the potential RBC, monocyte, and platelet contamination possible in light scatter gate determinations. This "immunosum" absolute lymphocyte count may then be compared to the mean absolute lymphocyte determination by light scatter. This provides a more thorough check of lymphocyte determination, than with the use of a CD45/CD14 reagent. All potential contaminating cell types are eliminated from this determination, not just the monocytes eliminated by CDC recommended CD45/CD14.

The end result is a patient summary report which contains all of the clinically relevant data which can be obtained from the larger CDC recommended 2-color panel. The advantages are faster and safer preparation of samples, higher sample through-put, reduced need for retesting, and more accurate data. In addition, since the sample preparation is simplified, and the analysis algorithm is essentially the same for each reagent, the result is a system which can be easily automated. This translates into substantial cost savings for the large clinical laboratory, and clinically relevant results which can be reported with a high level of confidence.

Automatic Selection of Cell Populations

The software component of the invention allows for the flow cytometer to collect and automatically analyze data, and generate reports for each sample at run time. The algorithm provides for the quality control of patient samples, reagents, and instrumentation.

For the automatic identification of cell populations, the software provides for predefined automatically generated gate detection regions. These detection regions may be positioned anywhere in two-parameter data space. After data acquisition from each sample, the software automatically attempts to detect a cell population within each defined region. This process of automated population identification is referred to as "autogating". The ability to autogate a cell population is a criterion for abnormal sample detection used in quality control. If the system is unable to successfully autogate the lymphocyte population, or any other population of interest, or if the lymphocyte peak is in an abnormal position, the sample is flagged for reanalysis.

Further sample quality control is obtained through several internal data checks. The lymphocyte count obtained from forward versus right angle light scatter is compared to "immuno-scatter" determination of NK$^+$B$^+$T cells. When the comparison falls out of range, the sample is flagged as suspect.

The software also monitors instrument quality control by tracking the sensitivity and stability of the flow cytometer. The system allows the user to enter, acquire, track and maintain instrument quality control data. The stability of the optical components of the system is monitored by measuring the mean and co-efficient of variance of the two light scatter parameters, and three fluorescent parameters of the instrument. The stability of the fluids system and absolute count capabilities are also quality controlled. The software also allows for tracking reagent quality control in a lot-specific manner. All quality control information is stored in the data base. The user may view or print Levy-Jennings charts for each of the quality control parameters, on a weekly or monthly basis, for a time interval specified by the user.

In addition to quality control reports, the software also generates system configuration, sample configuration, and patient test result reports. With regard to lymphocyte analysis, in particular, patient test results can include (1) absolute counts of total lymphocytes, T-cells, and $CD4^+$ T-cells; (2) a calculated CD4/CD8 ratio; and (3) $CD4^+$, $CD8^+$, and $CD3^+$ T-cells, B-cells, and NK-cells expressed as a percentage of the mean total lymphocytes. Flexible patient identification and comment fields are also a standard part of these reports. If a display of the actual data is desired, a cytogram or histogram may also be printed in a choice of formats. The related statistical results associated with any region or gates may also be printed.

The algorithm is adaptable to be applied to any data list displayed in any number of parameters. In its presently preferred embodiment, the algorithm works on any two parameter cytogram. The user specifies a detection region, which is typically rectangular, within which the software is to detect some cell subpopulation, represented by some cluster of data points. The software detects the cluster and specifies the cluster boundaries by drawing around the cluster a polygon, referred to as the autogate. In a preferred embodiment, the autogate is defined by 36 points. In addition, the user specifies, via an input parameter, how closely around the cluster to draw the 36 point polygon.

The algorithm is generally applicable to any cytogram. The algorithm uses image processing techniques to detect patterns within the user defined autogate zone (sensing zone). In a preferred embodiment, the cytogram data is stored in a 256 by 256 channel matrix. The algorithm transforms the 256 by 256 cytogram matrix according to a user specified parameter. If the parameter is 1 the cytogram matrix is left undisturbed (still a 256 by 256 matrix). If the parameter is 2, the algorithm applies a 2 by 2 convolution kernel (low pass filter) thus effectively reducing the cytogram to 128 by 128. The low pass filter has the effect of blurring the image so that details can be removed and large object (cell subpopulation) extraction can take place. In effect, this kernel transforms the 256 by 256 matrix to a 128 by 128. If the user defined parameter is 4, the matrix is transformed to 64 by 64 (a 4 by 4 convolution kernel is applied). The user defined parameter determines how fuzzy the transformed image is in relation to the 256 by 256 original and how tight the autogate is around a cell subpopulation. A parameter of 1 creates a very tight gate (the polygon is close to boundaries of the cell subpopulation) around a pattern on a cytogram. A parameter of 2 defines a looser gate around a cell subpopulation because the pattern within the autogate zone has been blurred. A parameter of 4 defines an even looser gate. The ability to tune the algorithm gives the researcher and the clinical technician great flexibility because the algorithm can be customized to specific needs.

The algorithm then finds the peaks within the user defined autogate zone (sensing region) on the transformed matrix. The algorithm scans the portion of the cytogram matrix within the autogate zone an element at a time. Edge matrix elements may be excluded in the scan. A region, which may typically be 3 elements by 3 elements, is formed around each matrix element. The edge matrix elements are included in the 3 by 3 region. The number of events, or data points, within the 3 by 3 region is calculated. The peak is determined to be the matrix element in the middle of the most intense 3 by 3 region, which is the region with the greatest number of events.

The algorithm then calculates the x and y coordinates of each of the 36 points defining the boundary of the autogate using a 10 degree radial projection algorithm, following radial projections from the gate peak at 10 degree intervals. The x, y coordinates for a boundary point are determined by scanning along a radial projection from the peak point. The boundary point is that point at which there is a minimum density of data points, usually 0. After the boundary points have been determined, it is desirable to recalculate the position of the peak as the average of the 36 points defining the autogate, and then to recalculate the 36 point autogate using the algorithm based on the new gate peak. The peak and autogate are recalculated because a detected peak may lie close to the edge of an autogate zone. The first time the 10 degree radial projection algorithm is applied, part of the calculated autogate boundary (some of the 36 autogate points) may lie outside the autogate zone. No clipping is applied the first time the 36 point autogate points are calculated. In order to "pull" the peak closer to the center of the autogate zone a new peak is calculated by averaging the values of the x and y coordinates of the points defining the first calculated autogate. The newly calculated peak is then used to recalculate a new 36 point autogate. The peak is pulled closer to the center since the algorithm follows the rule that the autogate must be inside the autogate zone (clipped within the autogate zone). The algorithm can be further extended by specifying to it via the use of the mini interpreted language to clip within the autogate zone or not to clip within the autogate zone. In addition, the user may define multiple overlapping autogate zones. This technique can be used, especially in flow-cytometry, to detect and distinguish overlapping cell populations. The autogate is smoothed with a 5 point average, by scanning each radial projection five times and using the average of the five measurements as the boundary point.

The data within the calculated autogate is then subtracted from the transformed cytogram matrix. The process of finding and subtracting peaks from the cytogram matrix may be repeated until a number of peaks have been found. In a preferred embodiment, the algorithm can find up to five peaks representing five cell subpopulations, within the same autogate zone.

The algorithm then evaluates which of the peaks is more pronounced or more important. The evaluation criteria are: (1) count of cells within the autogate, i.e. the autogate is more important if it has more cells in it (linear proportionality law); and (2) The gate is more important if it is closer to the center of the autogate zone (inverse square law). In a preferred embodiment, the density of each peak is divided by $r^2$, where r is the distance from the edge of the autogate zone. Thus, the most intense peak which is closest to the center of the autogate zone is selected, and peaks closer to the edge, which belong to cell populations other than the one being selected, are excluded.

The autogating capability of the present invention enables the selection of particular data subsets, or cell populations, to be made more accurately than previous gating methods, which specified a fixed rectangular gate region around the selected population. By forming a polygonal gate region around the population, which varies in configuration according to the density of the data points enclosed therein, the present invention provides higher certainty that all of the data points of a selected population are included in the gate, and that data points representing other populations are excluded. Furthermore, the ability to adjust the tightness of the autogate avoids the possibility that a boundary point generated by a radial projection from the peak point will be located inside the actual boundary. That is, it is possible that when a radial projection is scanned, a density minimum will be encountered which is located within the boundary of the selected population, rather than at the actual boundary. In this case, a false boundary point would be generated and some of the desired data points would be excluded by the resulting gate. This problem may be avoided by the choice of the convolution kernel which transforms the matrix. By fuzzing the matrix, such false density minima are eliminated.

The criteria outlined above are specific to a preferred implementation of the algorithm. The evaluation criteria can change from one algorithm implementation to the next. Therefore, one can extend this system by enabling the algorithm to read evaluation criteria from a file. The evaluation criteria can be easily changed with an editor. The algorithm will be language driven. A small language interpreter can be implemented to read, interpret and implement the algorithm's evaluation criteria. Further enhancements to this algorithm include the actual identification of specific cell subpopulations by using statistical fitting techniques to the cell subpopulations detected, for example, by fitting to a Gaussian distribution.

One preferred embodiment of the invention may be further understood by referring to the following pseudocode description of the autogate algorithms.

```
AUTOGATE ALGORITHM:
INPUT PARAMETERS:
Gate_Tightness (possible values 1,2,4).
Upper_Left_Autogate_Zone_X.
Upper_Left_Autogate_Zone_Y.
Lower_Right_Autogate_Zone_X.
Lower_Right_Autogate_Zone_Y.
Cytogram Matrix.
OUTPUT PARAMETERS:
A list of 36 (X,Y) points forming the autogate.

START ALGORITHM BODY.
Transform_Cytogram_To_Convolved_Cytogram( )
while (Find _Peak() )
do
        call Make_Autogate( )
        //
        // create a new peak by taking the average of the
        autogate
        //
        call Average_Autogate( )
        //
        // Pass the new peak along with the dimensions of the
        autogate zone.
        //
        call Make_Autogate( )
        Subtract the data within the final 36 point autogate
        from the Cytogram Matrix bounded by the Autogate
        Zone.
                Save the peak and the corresponding 36 point
                autogate.
                If the number of peaks detected is greater than
                5 then exit the while loop.
                endif
done
for each detected peak
do
        Score for current peak is
        number of cells within the autogate zone/the distance
        of the peak from the center of the autogate squared.
        if the score for the current peak is better than the
        best peak score found then
                replace the best peak score with the current
                score.
        endif


done
The 36 point autogate is the one corresponding to the peak
with the best score.
END ALGORITHM
```

```
PROCEDURE Transform_Cytogram_To_Convolved_Cytogram( )
INPUT PARAMETERS:
Cytogram Matrix
Size of the Convolved Cytogram Matrix
OUTPUT PARAMETERS:
Convolved Cytogram Matrix
START PROCEDURE BODY
if Gate_Tightness is 1 then
        for each element of the Convolved Matrix
            Convolved Cytogram Matrix element is equal to
            Cytogram Matrix element.
        end for
else if Gate_Tightness is 2 then
        for each element of the Convolved Matrix
            Convolved Cytogram Matrix element is equal to
            the sum of the elements of the 2 by 2 submatrix
            formed  when  grouping  the  Cytogram  Matrix
            elements in rows and columns of 2s
        end for
else if Gate_Tightness is 4 then
        for each element of the Convolved Matrix
            Convolved Cytogram Matrix element is equal to
            the sum of the elements of the 4 by 4 submatrix
            formed  when  grouping  the  Cytogram  Matrix
            elements in rows and columns of 4s
        end for
endif
END PROCEDURE
```

```
PROCEDURE Make_Autogate( )
INPUT PARAMETERS:
Cytogram matrix.
Upper Left (X,Y) coordinates of the Autogate Zone.
Lower Right (X,Y) coordinates of the Autogate Zone.
Peak in the autogate zone.
OUTPUT PARAMETERS:
The list of X and Y coordinates for the 36 point autogate
polygon.

START PROCEDURES BODY
do 10 degrees at a time (36 iterations total)
        Initialize the radial histogram to 0.
        do starting from the peak of the pattern
            Calculate the x and y coordinates for the next
            point of the radial
        histogram.
            if the next point is outside the autogate zone
            then
                Done with the current radial histogram.
                Go to next radial histogram (next 10
                degrees).
            endif
            Update the radial histogram.
        done
        Smooth the radial histogram with a 7 point average.
        Find the minimum of the smoothed radial histogram and
        save it as part of the  36 point boundary.
done
Smooth the 36 point boundary with a 5 point weighted
average.
Convert the 36 point boundary to x and y coordinates.
END PROCEDURE


PROCEDURE Average_Autogate( )
INPUT PARAMETERS:
A list of the (X,Y) coordinates of the 36 autogate points.
OUTPUT PARAMETERS:
The X average of the 36 autogate points.
The Y average of the 36 autogate points.

START PROCEDURE BODY
Form the sum of the X coordinates of the 36 autogate
points.
Form the sum of the Y coordinates of the 36 autogate
points.
Calculate the average of the X coordinates of the 36
autogate points.
Calculate the average of the Y coordinates of the 36
autogate points.
END PROCEDURE
```

```
PROCEDURE Find_Peak( )
INPUT PARAMETERS:
Cytogram matrix.
Upper Left coordinates and lower right coordinates of the
autogate zone.
OUTPUT PARAMETERS:
Peak in the autogate zone.
START PROCEDURE BODY
temporary sum of the Cytogram matrix elements covered by
the 3 by 3 scan matrix is 0.
do
        Form the sum of all Cytogram matrix elements covered
        by the 3 by 3 scan matrix.
        If the sum calculated is greater than the temporary
        sum then
                Current peak is designated as the (X,Y) pair in
                the middle of the Cytogram matrix elements
                covered by the 3 by 3 scan matrix.
        endif
        Move the 3 by 3 scan matrix to cover the next 3 by 3
        Cytogram matrix elements.
until the 3 by 3 scan matrix has covered the Cytogram
matrix.
END PROCEDURE
```

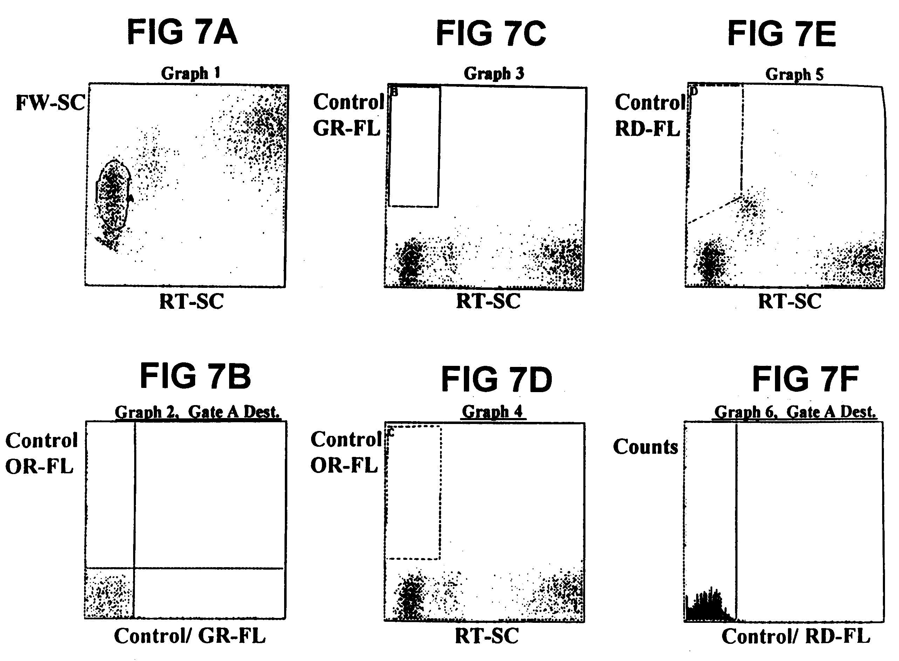

A more specific example of the algorithm, including the above-discussed autogating feature, will be given with reference to Figure 7A-7F, 8A-8D, 9A-9F, and 10.

In the operation of the algorithm, data acquisition and analysis are driven by reagent specific protocols and their association into panels. Panel specific properties define the operations performed and the reporting structures associated with the reagent panel. These properties include the reagent protocols used and, in some instances, the order in which the samples are acquired and analyzed.

In a fully automated operation, the control stained patient sample is run first. As shown in Figure 7A, each event is plotted on a graph. On this graph, the value of the right angle light scatter for the event is plotted along the x-axis or abscissa, and the value of the forward light scatter of the event is plotted along the y-axis or ordinate. A gate, referred to as a light scatter lymphocyte gate, is automatically set around the cluster of events on the graph that represent lymphocytes, and the total number of events inside that gate are counted to determine an absolute lymphocyte count.

The values of the green and orange fluorescent distributions for the cells inside the lymphocyte gate are plotted on a second graph, shown in Figure 7B, producing a green versus orange fluorescence cytogram. Also, as shown in Figure 7F, a histogram is produced showing, for each red fluorescence channel, the number of cells having measured red fluorescence in that channel. Four quadrant, statistical region markers are automatically set on the cytogram of Figure 7B, and consecutive negative and positive region markers are automatically set on the histogram of Figure 7F, based on user defined percent positive threshold values.

The non-specific fluorescence backgrounds, in each of three gate search regions to be used in the following two protocols, are also determined. This is done, as shown in Figures 7C, 7D and 7E, by plotting, for each event in the sample, the green, orange, and red fluorescence measurements, respectively, versus the right angle scattering measurements.

Either the second patient sample, referred to as the CD4/CD8/CD3 sample, or the third patient sample, referred to as the CD16/CD19/CD3 sample, may be run next.

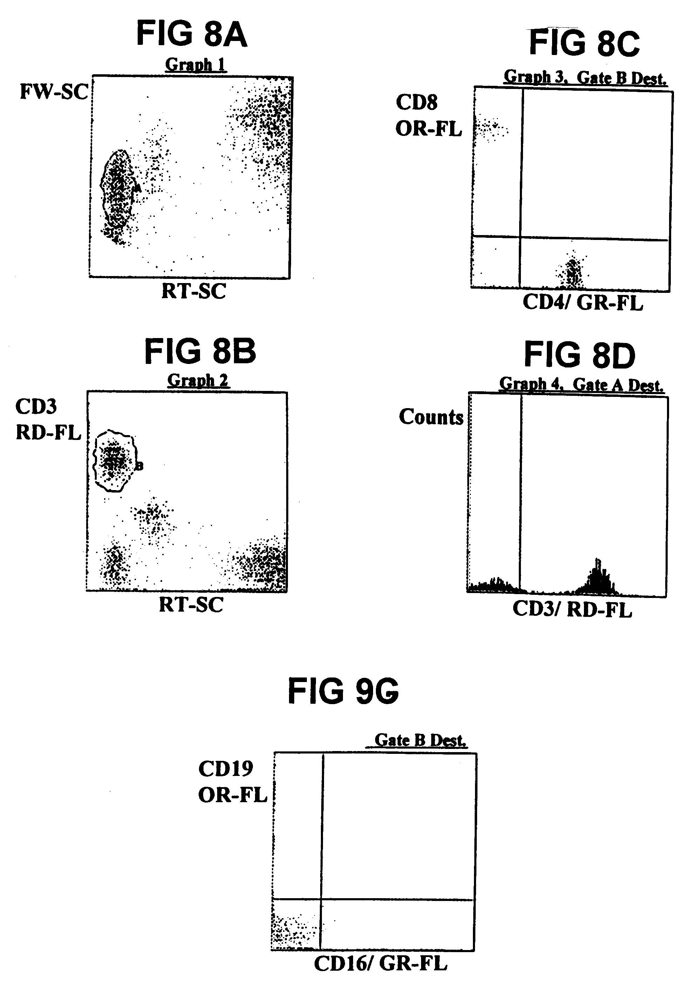

In the CD4/CD8/CD3 protocol, as in the control protocol, an absolute lymphocyte count is determined. With reference to Figure 8A, this is done by, first, plotting each event on a graph in which the right angle light scatter of the event is measured along the x-axis, and the forward angle light scatter of the event is measured along the y-axis, second, drawing an autogate around the cluster of events that represent lymphocytes, and third, counting the number of events inside that autogate.

A graph, shown in Figure 8B, is then made plotting, for each event in the second sample, the red fluorescence measurement of the event versus the right angle scattering measurement of the event. Then an autogate is drawn around the cluster of events on this graph that represent the T-cells, and the total number of cells inside that autogate is counted to provide an absolute count of the number of T-lymphocytes in the second patient sample.

To provide a check, a second absolute count of the T-cells is obtained from the second patient sample in a second manner. To do this, a histogram, shown in Figure 8D, is produced from the cells inside the lymphocyte gate of Figure 8A. For these cells, the histogram shows, for each red fluorescence channel, the number of cells having red fluorescence in that channel. All of these numbers that are contained in the positive region, as determined from Figure 7F are then summed to provide an absolute count of the number of lymphocytes that are CD3 positive. Since T-lymphocytes are CD3 positive and other lymphocytes are CD3 negative, this absolute count thus provides an absolute count of the number of T-lymphocytes in the second patient sample.

The CD3 gated cells --that is, the cells inside the autogate of Figure 8B-- are then plotted on another graph, shown in Figure 8C, to determine, first, the number of these cells that are also CD4 positive and CD8 negative, and second, the number of these cells that are also CD8 positive and CD4 negative. From the graph of Figure 8C, the CD4 positive CD8 positive CD3 cells may also be counted. More specifically, on the graph of Figure 8C, the orange fluorescence measurement of each of these cells (the CD3 gated cells) is plotted versus the green fluorescence measurement of each of these cells. The total number of cells having an orange fluorescence value above and a green fluorescence value below the background value, determined from Figure 7B, is counted to provide an absolute count of the CD8 positive CD4 negative cells; and the total number of cells having a green fluorescence value above and an orange fluorescence value below the threshold value, also determined from Figure 7B, is counted to provide an absolute count of the CD4 positive CD8 negative cells. The ratio of the latter number to the former number may then be calculated to indicate the ratio of CD4 positive cells to CD8 positive cells in the CD3 gated cells.

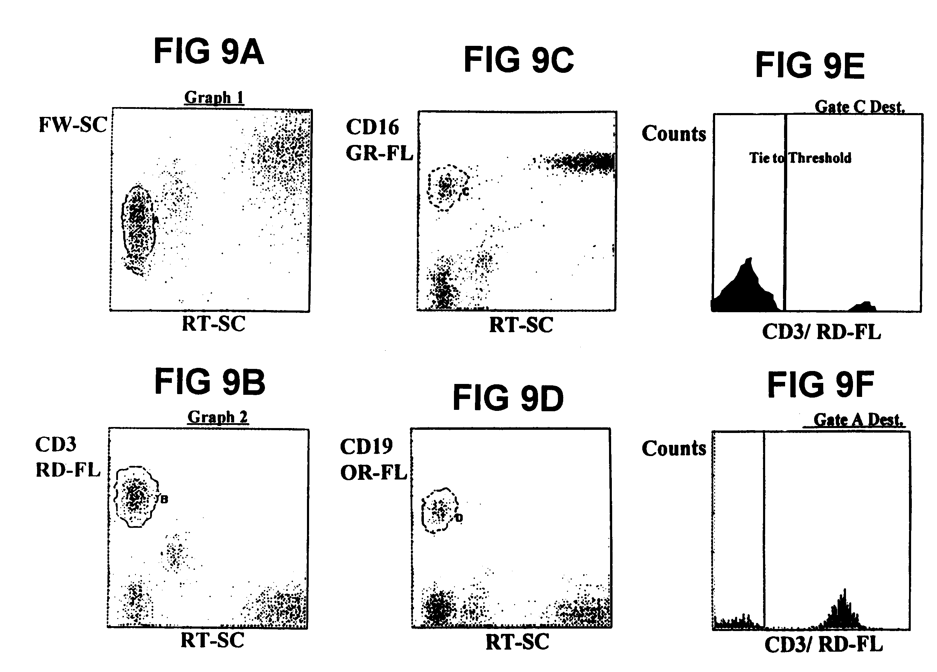

From the third patient sample, a third absolute lymphocyte count is determined also using a light scatter autogate. In particular, with reference to Figure 9A, each event is plotted on a graph, in which the right angle light scatter value for the event is plotted versus the forward light scatter value for the event. Then, an autogate is drawn around the cluster of events that represent lymphocytes, and the number of cells inside that autogate is counted.

This third patient sample is also used to determine a second absolute T-cell count. This is done by plotting all the events in the sample on a graph in which the x-axis represents the right angle scattering value for the event, and the y-axis represents the red fluorescence measurement for the event. An autogate is drawn around the cluster of these events, and the total number of cells inside that autogate is counted, producing an absolute count of the T-cells, which are lymphocyte cells that are CD3 positive.

A check is made of this T-cell count by sending the lymphocyte gate to a red fluorescence histogram, shown in Figure 9F. In particular, Figure 9F shows, for each red fluorescence channel, the number of cells in the lymphocyte gate of Figure 9A that have measured red fluorescence in that channel. All of these numbers that are above a threshold value are then summed to provide an absolute count of the lymphocyte cells that are also CD3 positive.

Next, absolute counts of the number of cells that are CD16 positive and CD19 positive are made. To do this, each cell in the sample is plotted on two graphs, shown in Figures 9C and 9D. In each of these graphs, the right angle scatter of the cell is plotted along the x-axis; and in the first graph, Figure 9C, the measured green fluorescence of the cell is plotted on the y-axis, and in the second graph, Figure 9D, the measured orange fluorescence of the cell is plotted on the y-axis. In the graphs of Figures 9C and 9D, autogates are then drawn around the clusters of cells that are CD16 positive and CD19 positive, respectively, and then each cell in each of these autogates is counted to determine, respectively, the total numbers of CD16 positive and CD19 positive cells in the sample.

After these counts are made, an absolute count of the number of cells in the sample that are both CD3 and CD16 positive is made. This is done by plotting the CD16 autogated cells onto a red fluorescent histogram, shown in Figure 9E. More specifically, Figure 9E shows, for each fluorescence channel, the number of cells in the CD16 autogate of Figure 9C that have measured red fluorescence in that channel. All of these numbers that are above a threshold value are summed to provide an absolute count of the number of cells that are CD3 positive and that were counted in the CD16 autogate. This count may then be subtracted from the total number of cells that were counted in the CD16 autogate to determine the number of cells in the sample that are CD16 positive and CD3 negative. These cells --CD16 positive and CD3 negative--are the NK cells in the sample.

This latter number may be estimated by means of an alternate procedure. In accordance with this alternate procedure, the CD3 gated cells of Figure 9B are sent to an orange fluorescence versus green fluorescence graph, as shown in Figure 9G. In particular, for each cell in the CD3 gate of Figure 9B, the green and orange fluorescence values of the cell are plotted, respectively, along the x-and y-axes of the graph of Figure 9G. The number of cells having green fluorescence values above and orange fluorescence values below the threshold values are counted, providing an absolute count of the number of cells that are both CD3 positive and CD16 positive. This number may then be subtracted from the total number of cells that are CD16 positive, as calculated from Figure 9C, to provide a count of the number of cells that are CD16 positive and CD3 negative.

The former of the two-above procedures for determining the number of CD16 positive and CD3 negative cells is preferred because the latter procedure may underestimate the total number of NK cells, particularly when the blood sample is taken from some HIV-positive patients. To elaborate, certain HIV-positive patient samples contain a fairly high frequency of low intensity CD16 positive cells that also bear CD3. These cells, which are not found in appreciable numbers in HIV-negative donor samples, are known to be CD8 cells and are responsible for ADCC. These ADCC cells, because of their low CD16 expression, might not be counted in the CD16 cells from the plot of Figure 9C.

From the above discussed data --and in particular, by summing the CD3 positive, the CD19 positive, and the CD16 positive CD3 negative lymphocyte counts-- a value, referred to as the immunosum, may be generated for the sum of the T-cells, B-cells and NK-cells. The use of the above-described algorithm provides such a value with greater purity, recovery and consistency than traditional light scatter gating. With the above-described algorithm, purity and recovery both approach 100% in most cases.

In addition, by virtue of the direct absolute counting nature of the system, it is possible to directly compare and combine results originating from other samples in the panel. This unique ability allows the use of the immunosum as a denominator for expressing the subsets as percentages of lymphocytes, without the need for an inaccurate and often inappropriate CD45/CD14 lymphocyte gate correction.

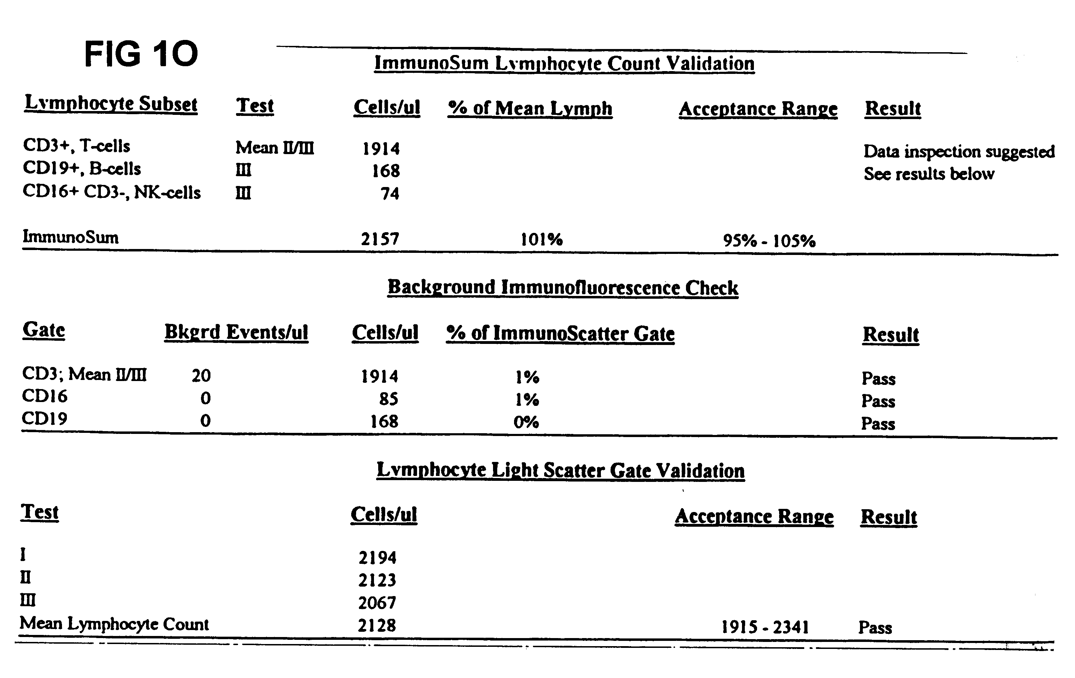

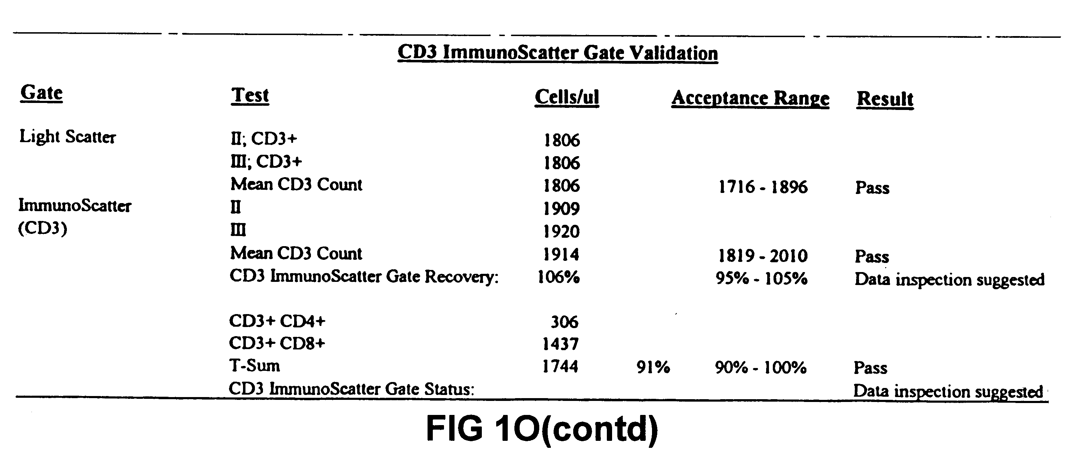

This ability also allows considerable cross checks to be made between the duplicate measurements obtained from the samples in the panel, and is the basis for a quality control report which may be generated as a result of the analysis. An example of such a report is given in Figure 10.

The quality control report is separated into several sections. Each section of the report checks areas in which automatic analysis of the data may require expert review, and helps to determine whether re-analysis of the data is warranted. If any section does not meet the defined criteria, a data inspection is suggested.

A background immunofluorescence check is performed in which the event counts found in the immunoscatter gates of the control sample are expressed as a percentage of the absolute counts obtained in the corresponding immunoscatter autogates of the positive specificities. If the percentages obtained are greater than 10%, then a data inspection is suggested.

The lymphocyte light scatter gates are cross checked. The mean of the triplicate determinations is calculated, and each lymphocyte gate count must fall within 10% of the mean to meet the criteria for acceptance.

The CD3 immunoscatter gates are validated as well, setting an acceptance range of 5% about the mean of the two duplicates. The CDC recommends a 3% range; however, no data has been offered to show improved results correlate with CD3 precision. The significance of this flag must be determined by the individual user.

This mean CD3 count value is further validated by comparison to the mean CD3 count obtained from the lymphocyte light scatter gates. The mean CD3 count from the immunoscatter autogates is expressed as a percentage of the mean CD3 count obtained from the light scatter gates. This CD3 immunoscatter gate recovery must fall between 95 and 105%.

Additional CD3 gate validation is achieved by comparison to the T-sum. The T-sum is determined by summing the $CD3^+CD4^+CD8^-$ and $CD3^+CD8^+CD4^-$ counts obtained from the CD4 vs CD8 cytogram. The $CD4^+CD8^+$ events are excluded from the calculation, thus providing a check on compensation as well. The T-sum is expressed as a percentage of the mean CD3 count obtained from the immunoscatter autogates. This value must fall between 90 to 100% for the result to be considered acceptable.

The final quality control check performed has with experience proven to be, overall, the most informative. The immunosum is validated against the mean, light scatter determined lymphocyte count. The immunosum is expressed as a percentage of the mean lymph count from light scatter. An acceptance range of 95 to 105% is used for the validation.

The examples provided herein exemplify a preferred embodiment of the invention. Those skilled in the art will understand from the description that many further applications, other than those specifically exemplified, are within the scope of invention.

## Claims

1. A method of selecting a subset of data from a matrix representing a data list comprising:
   (a) defining within the matrix a search region containing the subset;
   (b) locating a maximum density of data points within the search region;
   (c) locating a series of minimum densities of data points which define a boundary surrounding the maximum; and

(d) isolating the data points within the region surrounded by the boundary points.

2. A method of selecting from a matrix of data points representing cells, a set of data points representing a population of cells comprising:

(a) specifying within the matrix, a search region which contains the population;

(b) locating within the search region, a peak point which represents a maximum density of data points within the population;

(c) locating a series of boundary points surrounding the peak point which represent minimum densities of data points surrounding the peak point;

(d) generating a polygon defined by the boundary points surrounding the data points representing the population.

3. The method of Claim 2, wherein the peak point is located by dividing the search region into discrete subregions and determining which subregion includes a maximum density of data points.

4. The method of Claim 3, wherein the subregions are grouped in 3 X 3 blocks and the peak point is located by determining which block includes the subregion with the maximum density of data points.

5. The method of Claim 2, wherein the boundary points are located by sequentially determining the minimum density of data points on successive radial projections from the peak point.

6. The method of Claim 5 wherein the radial projections are at radial intervals of 10°.

7. A method of isolating from a flow cytometry cytogram matrix, a set of population data points corresponding to a specific population of cells for further analysis, comprising:

(a) defining a search region, within the cytogram matrix, which includes the population data;

(b) dividing the search region into discrete subregions;

(c) determining the density of population data points within each subregion;

(d) locating a peak point in the center of a subregion having the highest density of data points;

(e) determining a point of minimum density along each of a series of radial projections from the peak point;

(f) locating a boundary point at each of the minimum density points;

(g) defining a polygon which connects the boundary points and surrounds the population data points; and

(h) subtracting the population data within the polygon from the cytogram matrix data.

8. In a flow cytometry system for optically analyzing a sample in which data points representing cells in the sample are aggregated in a matrix based on selected optical parameters, a method of isolating a group of data points representing cells in an identified population, comprising:

(a) specifying within the matrix, a search region which contains the identified population;

(b) locating within the search region, a peak point which represents a maximum density of data points within the population;

(c) locating a series of boundary points surrounding the peak point which represent minimum densities of data points surrounding the peak point;

(d) generating a polygon defined by the boundary points; and

(e) subtracting from the matrix the data points within the polygon.

9. Apparatus arranged to operate the method of any one of claims 1 to 9.

10. A flow cytometry apparatus for producing an absolute CD4 count using three coloured reagents.

**FIG 1**

3- COLOR RED FILTER

ORANGE DICHROIC MIRROR

COLLIMATOR LENS

2- COLOR RED FILTER

GREEN DICHROIC MIRROR

GREEN PASS FILTER

FLUORESCENCE PINHOLE

BEAM SPLITTER

RIGHT ANGLE OBJ

FLOW CELL

LASER FOCUSING LENS

ORANGE FILTER

DETECTOR LENS -2

ORANGE DETECTOR

GREEN FILTER

DETECTOR LENS -1

GREEN DETECTOR

90° SCATTER PINHOLE

LASER PASS FILTER

90° SCATTER DETECTOR

90°

FORWARD DIFFUSER  FORWARD DIFFUSER

OBJ LENS

OPTICAL FIBER CABLE

ART ION LASER
λ-488nm 15mW

BLECHER BAR

FORWARD DETECTOR

BEAM SELECT FILTER

2- COLOR OPTICS

FORWARD PINHOLE

EP 0 633 462 A2

# FIG 2

FW-SC

RT-SC

Control-PE

## Auto Region Set

Control-FITC

## RESULTS

**Absolute Lymph**

EP 0 633 462 A2

**FIG 3**

RD-FL / RT-SC

GR-FL / RT-SC

OR-FL / RT-SC

## RESULTS

**Autogate Region Fluor Bkgrd:**

**RT-SC vs. GR-FL**
**RT-SC vs. OR-FL**
**RT-SC vs. RD-FL**

EP 0 633 462 A2

FIG 4

CD3-CyP

RT-SC

CD8-PE

CD4-FITC

FW-SC

RT-SC

RESULTS

Absolute Lymph

Absolute CD3+ T-cells

Absolute CD4+ T-cells

Absolute CD8+ T-cells

CD4/CD8 Determination

EP 0 633 462 A2

**FIG 5**

EP 0 633 462 A2

RESULTS

Absolute Lymph

Absolute CD3+ T-cells

CD3+CD16+ Determination

# FIG 6

CD16-FITC

RT-SC

CD19-PE

RT-SC

EP 0 633 462 A2

## RESULTS

Absolute CD19+ B-cells

Absolute CD16+CD3-  NK-cells

**FIG 7A**

Graph 1

FW-SC

RT-SC

**FIG 7C**

Graph 3

Control
GR-FL

RT-SC

**FIG 7E**

Graph 5

Control
RD-FL

RT-SC

**FIG 7B**

Graph 2, Gate A Dest.

Control
OR-FL

Control/ GR-FL

**FIG 7D**

Graph 4

Control
OR-FL

RT-SC

**FIG 7F**

Graph 6, Gate A Dest.

Counts

Control/ RD-FL

EP 0 633 462 A2

EP 0 633 462 A2

# FIG 8A

Graph 1

FW-SC

RT-SC

# FIG 8B

Graph 2

CD3
RD-FL

RT-SC

# FIG 8C

Graph 3, Gate B Dest.

CD8
OR-FL

CD4/ GR-FL

# FIG 8D

Graph 4, Gate A Dest.

Counts

CD3/ RD-FL

# FIG 9G

Gate B Dest.

CD19
OR-FL

CD16/ GR-FL

25

# FIG 9A

Graph 1

FW-SC

RT-SC

# FIG 9C

CD16
GR-FL

RT-SC

# FIG 9E

Counts

Gate C Dest.

Tie to Threshold

CD3/ RD-FL

# FIG 9B

Graph 2

CD3
RD-FL

B

RT-SC

# FIG 9D

CD19
OR-FL

D

RT-SC

# FIG 9F

Counts

Gate A Dest.

CD3/ RD-FL

EP 0 633 462 A2

# FIG 10

## ImmunoSum Lymphocyte Count Validation

| Lymphocyte Subset | Test | Cells/ul | % of Mean Lymph | Acceptance Range | Result |
|---|---|---|---|---|---|
| CD3+, T-cells | Mean II/III | 1914 | | | Data inspection suggested |
| CD19+, B-cells | III | 168 | | | See results below |
| CD16+ CD3-, NK-cells | III | 74 | | | |
| ImmunoSum | | 2157 | 101% | 95% - 105% | |

## Background Immunofluorescence Check

| Gate | Bkgrd Events/ul | Cells/ul | % of ImmunoScatter Gate | Result |
|---|---|---|---|---|
| CD3; Mean II/III | 20 | 1914 | 1% | Pass |
| CD16 | 0 | 85 | 1% | Pass |
| CD19 | 0 | 168 | 0% | Pass |

## Lymphocyte Light Scatter Gate Validation

| Test | Cells/ul | Acceptance Range | Result |
|---|---|---|---|
| I | 2194 | | |
| II | 2123 | | |
| III | 2067 | | |
| Mean Lymphocyte Count | 2128 | 1915 - 2341 | Pass |

EP 0 633 462 A2

## CD3 ImmunoScatter Gate Validation

| Gate | Test | Cells/ul | | Acceptance Range | Result |
|---|---|---|---|---|---|
| Light Scatter | II; CD3+ | 1806 | | | |
| | III; CD3+ | 1806 | | | |
| | Mean CD3 Count | 1806 | | 1716 - 1896 | Pass |
| ImmunoScatter | II | 1909 | | | |
| (CD3) | III | 1920 | | | |
| | Mean CD3 Count | 1914 | | 1819 - 2010 | Pass |
| | CD3 ImmunoScatter Gate Recovery: | 106% | | 95% - 105% | Data inspection suggested |
| | CD3+ CD4+ | 306 | | | |
| | CD3+ CD8+ | 1437 | | | |
| | T-Sum | 1744 | 91% | 90% - 100% | Pass |
| | CD3 ImmunoScatter Gate Status: | | | | Data inspection suggested |

# FIG 1O(contd)

EP 0 633 462 A2